# EUROPÄISCHE PATENTANMELDUNG

(11) **EP 4 847 587 A1**
(43) Veröffentlichungstag der Anmeldung: **02.12.2026**
(21) Anmeldenummer: 26180988.3
(22) Anmeldetag: 26.05.2026
(51) Int. Cl.: C10L 3/08

(54) **VERFAHREN UND REAKTORSYSTEM ZUR KATALYTISCHEN METHANISIERUNG EINES KOHLENDIOXID UND WASSERSTOFF ENTHALTENDEN EDUKT-GASES**

(30) Priorität: 28.05.2025 DE 102025120849
(71) Anmelder: DWE GmbH, 94469 Deggendorf (DE)
(72) Erfinder: Pichler, Sebastian, 81927 München (DE); Schuhbauer, Christian, 94336 Hunderdorf (DE)
(74) Vertreter: Sturm, Christoph

(57) **Zusammenfassung**

Verfahren zur katalytischen Methanisierung eines Kohlendioxid und Wasserstoff enthaltenden Edukt-Gases (11), mit folgenden Schritten: a) Bereitstellen des Edukt-Gases (11), b) katalytische Teil-Methanisierung des Edukt-Gases (11) in einer ersten Reaktorstufe (12), deren Katalysatorschüttung von einem ersten Wärmeträger gekühlt wird, c) Abkühlen eines aus der ersten Reaktorstufe (11) ausströmenden ersten Produkt-Gases (15) unterhalb des Taupunkts von Wasser und Ableiten des kondensierten Wassers, d) Aufheizen des ersten Produkt-Gases (15), aus welchem das im Schritt c) auskondensierte Wasser abgeleitet wurde, e) katalytische End-Methanisierung des in Schritt d) aufgeheizten ersten Produkt-Gases (15) in einer zweiten Reaktorstufe (20), deren Katalysatorschüttung von einem zweiten Wärmeträger gekühlt wird, f) Abkühlen eines aus der zweiten Reaktorstufe (20) ausströmenden zweiten Produkt-Gases und Ableiten des hierbei kondensierten Wassers. Zur Gewinnung eines zweiten Produkt-Gases (23), welches einen Kohlendioxidanteil von maximal 100 ppm(v) und einen Wasserstoffanteil von maximal 2,0 Volumen-% aufweist, wird in Schritt a) ein Edukt-Gas (11) bereitgestellt wird, das in Bezug auf die Reaktion von Kohlendioxid mit Wasserstoff einen überstöchiometrischen Wasserstoffanteil aufweist, wobei in Schritten b) und e) in der ersten und zweiten Reaktorstufe (12, 20) die jeweilige Katalysatorschüttung als Katalysatormaterial Ruthenium aufweist, und wobei in Schritten b) und e) innerhalb der ersten und zweiten Reaktorstufe (12, 20) jeweils eine Wärmeträger-Temperatur bereitgestellt wird, die zwischen 180°C und 250°C liegt.

## Beschreibung

Die Erfindung betrifft ein Verfahren zur katalytischen Methanisierung eines Kohlendioxid und Wasserstoff enthaltenden Edukt-Gases. Des Weiteren betrifft die Erfindung ein Reaktorsystem zur katalytischen Methanisierung eines Kohlendioxid und Wasserstoff enthaltenden Edukt-Gases.

Vor dem Hintergrund endlicher Erdgasvorkommen gibt es ein steigendes Interesse an der Herstellung eines Erdgassubstituts. Das Erdgassubstitut wird auch als Substitute Natural Gas (SNG) oder als Synthetic Natural Gas (SNG) oder als Austauschgas bezeichnet. Erdgassubstitut enthält im Wesentlichen Methan und geringe Anteile anderer Gase. Erdgassubstitut kann auf Basis von Kohle oder auf Basis von anderen kohlenstoffhaltigen Stoffen wie Abfall oder Biomasse oder auf Basis von über Synthesegas hergestellt werden. Das zur Herstellung von Erdgassubstitut verwendete Edukt-Gases kann eine Mischung aus Kohlenstoffmonoxid CO und/oder Kohlendioxid CO₂, Wasserstoff H₂ und gegebenenfalls anderen Bestandteilen wie Wasser sein.

Die Methanisierung einer Mischung aus Kohlenmonoxid CO und Wasserstoff H₂ beruht auf folgender chemischer Reaktion:

CO + 3 H₂ ↔ CH₄ + H₂O ΔHR₀ = -206 kJ/mol (1).

Die Methanisierung einer Mischung aus Kohlendioxid CO₂ und Wasserstoff H₂ beruht auf folgender chemischer Reaktion:

CO₂ + 4 H₂ ↔ CH₄ + 2 H₂O ΔHR₀ = -165 kJ/mol (2).

Bei der Methanisierung können Nebenreaktionen ablaufen, und zwar eine Wassergas-Shift-Reaktion:

CO + H₂O ↔ H₂ + CO₂ ΔHR₀ = -41 kJ/mol (3).

sowie eine Boudouard-Reaktion, bei welcher sich Kohlenmonoxid zu Kohlenstoff und Kohlendioxid zersetzt:

2 CO ↔ C + CO₂ ΔHR₀ = -173 kJ/mol (4).

Für die Methanisierung gemäß den obigen Reaktionsgleichungen (1) und (2) werden in der Regel Trägerkatalysatoren mit einer katalytisch aktiven Beschichtung verwendet, wobei als katalytisch aktive Beschichtung in der Regel Nickel Ni und als Trägermaterial in der Regel Titanoxid TiO₂ oder Aluminiumoxid Al₂O₃ genutzt werden. WO 2019/ 154 982 A1 oder CN 115 970 746 A offenbaren diesbezüglichen Stand der Technik.

Die obigen Reaktionen stehen miteinander im Gleichgewicht, wobei sich die Lage der Gleichgewichte verändern lässt. Die Lage des Gleichgewichts hängt von Verfahrensparametern wie Druck, Temperatur und Konzentrationen der Reaktionskomponenten ab. Bei den obigen Reaktionsgleichungen (1) und (2) ergibt sich eine Volumenabnahme auf der Produktseite. Die Methanisierungen gemäß den Reaktionsgleichungen (1) und (2) sind zudem exotherm und molzahlreduzierend. Dies führt dazu, dass ein hoher Druck und eine niedrige Temperatur das Reaktionsgleichgewicht auf die Produktseite verschieben. Bei einem hohen Druck wird der Einfluss auf das Reaktionsgleichgewicht jedoch geringer. Die Reaktionstemperatur ist nach unten durch die Mindestarbeitstemperatur des zur Methanisierung genutzten Katalysators begrenzt. Eine steigende Reaktionstemperatur erhöht zwar die Reaktionsgeschwindigkeit, nachteilig ist jedoch eine Begrenzung des Umsatzes infolge einer Verschiebung des Reaktionsgleichgewichtes auf die Eduktseite und eine verstärkte Alterung des Katalysators.

Zur optimalen Ausnutzung der reaktionstechnischen Randbedingungen werden oftmals mindestens zwei Reaktionsstufen verwendet. Dabei wird eine erste Reaktionsstufe in der Regel mit einer höheren Temperatur und eine zweite Reaktionsstufe mit einer niedrigeren Temperatur betrieben, um den Umsatz zu maximieren. Nicht reagierte Bestandteile können gemäß EP 3 018 190 A1 vom Produktgas abgetrennt und in den Prozess zurückgeführt werden.

Das Reaktionsgleichgewicht lässt sich nach DE 10 2018 113 737 A1 weiter auf die Produktseite verschieben, indem Wasser H₂O aus dem Reaktionssystem entfernt wird. Eine zu starke Wasserentfernung begünstigt jedoch die Bildung von festem Kohlenstoff auf der Katalysatoroberfläche durch die Boudouard-Reaktion gemäß obiger Gleichung (4).

Je nach Anforderung an das Produktgas kann das Reaktionsgleichgewicht auch durch die Zusammensetzung des Edukt-Gases beeinflusst werden. Gemäß DE 29 40 334 A1 wird die Methanisierung eines Synthesegases unter Wasserstoffüberschuss durchgeführt, wobei der überschüssige Wasserstoff aus dem Produktstrom abgetrennt und in die Methanisierung zurückgeführt wird. Die nicht reagierten Gasbestandteile werden mit großem Aufwand von dem Zielprodukt Methan CH₄ getrennt und in den Prozess zurückgeführt.

Das bei der Methanisierung gewonnene Erdgassubstitut hat im Wesentlichen die gleichen Eigenschaften wie fossil gewonnenes Erdgas und kann daher durch die bereits bestehende Erdgasinfrastruktur transportiert und genutzt werden. Zum Transport von Erdgassubstitut in großen Mengen können entweder Rohrleitungsnetzwerke wie Pipelines genutzt werden, oder das Gas wird durch starke Abkühlung unterhalb des Siedepunkts von Methan (ca. -162°C) verflüssigt und anschließend mit Fahrzeugen wie z.B. Lastkraftwagen oder Schiffen transportiert. Diese Form des Gases ist allgemein als Liquefied Natural Gas (LNG) bekannt.

Für die Einspeisung von Erdgassubstitut in Gasnetzwerke gelten andere Vorgaben wie an die Gasqualität wie für LNG. Während bei Gasnetzwerken der Gasnetzbetreiber die erforderliche Einspeisegasqualität so festlegt, dass das Gas konstante brenntechnische Eigenschaften hat, steht bei LNG ein möglichst niedriger Anteil an Kohlendioxid im Vordergrund. Der Grund dafür ist, dass Kohlendioxid unter Umgebungsdruck bereits bei -79°C resublimiert und damit als Feststoff ausfällt, was den Verflüssigungsprozess stört und zu Anlagenabschaltungen führen kann, um die Ablagerungen zu entfernen. Als Grenzwert gilt üblicherweise, dass maximal 100 ppm(v), insbesondere maximal 50 ppm(v), Kohlendioxidanteil im zu verflüssigenden Gas enthalten sein darf. Außerdem ist bei der Methanisierung zur Herstellung von LNG aus Effizienzgründen der Verflüssigungsanlage ein niedriger H₂-Anteil von kleiner 2,0 Vol.-% anzustreben.

Im Bereich der fossilen Erdgasgewinnung und LNG-Produktion ist es durchaus üblich, dass die natürlichen Gasvorkommen einen erheblichen Anteil an Kohlendioxid enthalten. Um den Kohlendioxidanteil auf den zulässigen Grenzwert zu reduzieren, werden verschiedene Abtrennverfahren angewandt. Zu den etablierten Verfahren zählen die Aminwäsche, KOH-Wäsche, Methanolwäsche, Adsorption, Membranverfahren und das Controlled Freeze-Verfahren. Diese Verfahren sind erprobt und zuverlässig, erfordern jedoch einen entsprechenden apparativen und betrieblichen Aufwand.

CN 103 706 373 A offenbart einen Niedrigtemperatur-Katalysator auf Nickelbasis mit Al₂O₃ als Trägermaterial. Dieser Niedrigtemperatur-Katalysator ist besonders geeignet zur Methanisierung von Synthesegas aus der Kohlevergasung. Ein solches Synthesegas hat im Allgemeinen einen geringen Anteil an Kohlenstoffoxiden und hohe Anteile an CH₄ und H₂. Das Edukt-Gas gemäß CN 103 706 373 A enthält 0,2% bis 2,0% CO₂, 2,0 bis 23% H₂. Der Rest besteht aus 85% bis 97,8% CH₄. Eine Anwendung auf ein Edukt-Gas mit gänzlich anderer Zusammensetzung, wie beispielsweise ein Edukt-Gas mit im Wesentlichen ausschließlich CO₂ und H₂ in etwa stöchiometrischer Zusammensetzung, wird nicht beschrieben.

DE 10 2018 113 735 A1 und DE 10 2004 040 472 A1 offenbaren weiteren Stand der Technik.

Es besteht Bedarf, ein Erdgassubstitut herzustellen, welches ohne weitere Maßnahmen zur Kohlendioxidabscheidung die Gasqualitätsanforderungen von Erdgasverflüssigungsanlagen erreicht. Konkret bedeutet dies, dass ein Erdgassubstitut hergestellt werden soll, welches einen Kohlendioxidanteil von maximal 100 ppm(v), insbesondere von maximal 50 ppm(v), und einen Wasserstoffanteil von maximal 2,0 Vol.-% enthält. Hiervon ausgehend liegt der vorliegenden Erfindung die Aufgabe zugrunde, ein neuartiges Verfahren und Reaktorsystem zur katalytischen Methanisierung eines Kohlendioxid und Wasserstoff enthaltenden Gases zu schaffen. Diese Aufgabe wird durch ein Verfahren zur katalytischen Methanisierung eines Kohlendioxid und Wasserstoff enthaltenden Gases nach Anspruch 1 und durch ein Reaktorsystem nach Anspruch 16 gelöst.

Das erfindungsgemäße Verfahren zur katalytischen Methanisierung eines Kohlendioxid und Wasserstoff enthaltenden Edukt-Gases umfasst die folgenden Schritte: Schritt a): Bereitstellen des Kohlendioxid und Wasserstoff enthaltenden Edukt-Gases. Schritt b): katalytische Teil-Methanisierung des Edukt-Gases in einer ersten Reaktorstufe, deren Katalysatorschüttung von einem ersten Wärmeträger gekühlt wird. Schritt c): Abkühlen eines aus der ersten Reaktorstufe ausströmenden ersten Produkt-Gases auf eine Temperatur unterhalb des Taupunkts von Wasser und Ableiten des hierbei kondensierenden Wassers. Schritt d): Aufheizen des ersten Produkt-Gases, aus welchem das im Schritt c) auskondensierte Wasser abgeleitet wurde. Schritt e) katalytische End-Methanisierung des in Schritt d) aufheizten ersten Produkt-Gases in einer zweiten Reaktorstufe, deren Katalysatorschüttung von einem zweiten Wärmeträger gekühlt wird. Schritt f): Abkühlen eines aus der zweiten Reaktorstufe ausströmenden zweiten Produkt-Gases auf eine Temperatur unterhalb des Taupunkts von Wasser und Ableiten des hierbei kondensierten Wassers.

Zur Gewinnung des zweiten Produkt-Gases mit einem Kohlendioxidanteil von maximal 100 ppm(v), insbesondere von maximal 50 ppm(v), und mit einem Wasserstoffanteil von maximal 2,0 Vol.-% wird in Schritt a) ein Edukt-Gas bereitgestellt, das in Bezug auf die Reaktion von Kohlendioxid mit Wasserstoff einen überstöchiometrischen Wasserstoffanteil aufweist, ferner weist in Schritten b) und e) in der ersten und in der zweiten Reaktorstufe die jeweilige Katalysatorschüttung als Katalysatormaterial Ruthenium auf, ferner wird in Schritten b) und e) innerhalb der ersten Reaktorstufe und innerhalb der zweiten Reaktorstufe jeweils eine Wärmeträger-Temperatur bereitgestellt, die zwischen 180°C und 250°C liegt.

Durch den Überschuss an Wasserstoff verschiebt sich das Reaktionsgleichgewicht auf die Produktseite. Der Umsatz von Kohlendioxid CO₂ wird begünstigt. Die Umsetzung wird durch die Verwendung von Ruthenium als katalytisch aktive Substanz begünstigt, da dieser Katalysator schon bei niedrigen Temperaturen ab ca. 180°C katalytisch wirksam ist. Zusätzlich werden die Wärmeträger-Temperaturen gezielt eingestellt. Durch die Kombination zumindest der obigen Maßnahmen kann in überraschender Weise die gewünschte Gasqualität, die sich durch einen Kohlendioxidanteil von maximal 100 ppm(v), insbesondere maximal 50 ppm(v), und durch einen Wasserstoffanteil von maximal 2,0 Vol.-% auszeichnet, sicher erreicht werden, und zwar mit geringem vorrichtungsseitigem Aufwand. Ein wesentlicher Vorteil der Erfindung ist vor allem die Erhöhung des erzielbaren Umsatzes aufgrund der hohen Aktivität des Ruthenium Katalysators, was in Kombination mit den gewählten Reaktionsbedingungen und dem leichten Wasserstoffüberschuss zu einer gewünschte Gasqualität führt, die unter Berücksichtigung einer anschließenden Gastrocknung den Anforderungen an Produktionsanlagen für Flüssigerdgas entspricht. Aus dem Stand der Technik bekannte Verfahren erzielen nicht die Anforderung an die gewünschte Gasqualität und müssen insbesondere mit einer gesonderten nachgeschalten Kohlendioxid-Abtrennungsanlage ausgestattet werden. Dies stellt einen erheblichen Kostennachteil von aus dem Stand der Technik bekannte Verfahren gegenüber dem erfindungsgemäßen Verfahren dar.

Vorzugsweise wird in Schritt b) in der ersten Reaktorstufe und in Schritt e) in der zweiten Reaktorstufe jeweils eine Katalysatorschüttung verwendet, die einen Rutheniumanteil zwischen 0,1 Masse-% und 5,0 Masse-% aufweist. Befindet sich der Massenanteil von Ruthenium im beanspruchten Bereich, so kann die gewünschte Gasqualität sicher erreicht werden.

Vorzugsweise wird in Schritt a) ein Edukt-Gas bereitgestellt, welches in Bezug auf die Reaktion von Kohlendioxid mit Wasserstoff einen um bis zu 0,5 % höheren Wasserstoffanteil aufweist als eine stöchiometrische Mischung aufweist. Mit einem solchen Edukt-Gas kann die gewünschte Gasqualität sicher erreicht werden.

Vorzugsweise wird in Schritt a) ein Edukt-Gases bereitgestellt, welches im Wesentlichen ausschließlich Kohlendioxid und Wasserstoff besteht, derart, dass die Summe aus dem Kohlendioxidanteil und dem Wasserstoffanteil im Edukt-Gas mindestens 99 Volumen-%, beträgt. Alternativ besteht das in Schritt a) bereitgestellte Edukt-Gas im Wesentlichen aus Kohlendioxid, Kohlenmonoxid und Wasserstoff, derart, dass die Summe aus dem Kohlendioxidanteil, Kohlenmonoxidanteil und dem Wasserstoffanteil im Edukt-Gases mindestens 99 Volumen-% des Edukt-Gases beträgt, wobei maximal 5 Volumen-% des Kohlendioxids durch Kohlenmonoxid ersetzt sind. Das erfindungsgemäße Verfahren eignet sich besonders zur Methanisierung eines Edukt-Gases, welches im Wesentlichen ausschließlich aus Kohlendioxid und Wasserstoff besteht.

Vorzugsweise liegt in Schritt b) in der ersten Reaktorstufe und in Schritt e) in der zweiten Reaktorstufe die jeweilige Wärmeträger-Temperatur zwischen 200°C und 230°C oder zwischen 200°C und 220°C. Dieser Temperaturbereich für die jeweilige Wärmeträger-Temperatur ist besonders geeignet, um die gewünschte Gasqualität sicher zu erreichen. Insbesondere ist dieser Temperaturbereich optimal hinsichtlich des erzielbaren Umsatzes, der erforderlichen Anspringtemperatur der Reaktion und des Vermeidens von unerwünschten Nebenreaktionen bei zu tiefen Wärmeträger-Temperaturen. Die Wärmeträger-Temperaturen zwischen 200°C und 230°C oder zwischen 200°C und 220°C haben zahlreiche Vorteile. Bei der Verwendung von Siedewasser als Wärmeträger ergeben sich kleinere Betriebsdrücke auf der Wärmeträgerseite. Dadurch ergeben sich kleinere Wandstärken aller vom Siedewasser berührten Teile, günstigere Werkstoffe oder längere Katalysatorstandzeiten.

Vorzugsweise weist das nach Schritt c) vorliegende erste Produkt-Gas einen Wasserdampfgehalt von maximal 30 Volumen-%, vorzugsweise einen Wasserdampfgehalt von zwischen 5 Volumen-% und 15 Volumen-%, auf. Ein solcher Wasserdampfgehalt führt einerseits zu einer günstigen Verschiebung des Reaktionsgleichgewichtes zur Produktseite, andererseits ist noch genügend Wasser im Reaktionsgas enthalten zum Verhindern von unerwünschten Kohlenstoffablagerungen. Die gewünschte Gasqualität kann sicher erreicht werden.

Vorzugsweise werden in Schritten b) und e) als jeweilige Reaktorstufe jeweils eine isotherme Rohrbündel-Reaktorstufe verwendet, die jeweils ein Bündel aus mit der Katalysatorschüttung befüllten Reaktionsrohren aufweist, die vom Wärmeträger gekühlt werden. Rohrbündelreaktoren sind erprobte Reaktortypen und erlauben das sichere Abführen von Reaktionswärme von insbesondere stark exothermen Reaktionen. Die gewünschte Gasqualität kann in Rohrbündel-Reaktorstufen sicher erreicht werden.

Vorzugsweise wird in Schritt b) in der ersten Reaktorstufe und/oder in Schritt e) in der zweiten Reaktorstufe, vorzugsweise zumindest oder ausschließlich in Schritt b) in der ersten Reaktorstufe, das in die jeweilige Reaktorstufe einströmende Gas in mindestens zwei Teilströme aufgeteilt, die an unterschiedlichen Positionen in die jeweilige Katalysatorschüttung eingeleitet werden. Durch die gestufte Zugabe des jeweils einströmenden Gases in den Reaktionsraum der jeweiligen Reaktorstufe kann ein sogenannter Hotspot sicher kontrolliert werden. Ein Durchgehen der Reaktion wird so sicher verhindert. Dies dient letztendlich auch dazu, die gewünschte Gasqualität sicher zu erreichen.

Vorzugsweise wird in Schritt b) in der ersten Reaktorstufe und/oder in Schritt e) in der zweiten Reaktorstufe, vorzugsweise zumindest oder ausschließlich in Schritt b) in der ersten Reaktorstufe, eine Katalysatorschüttung verwendet, die mindestens zwei Katalysatorschichten unterschiedlicher Aktivität aufweist, wobei die Aktivität einer ersten Katalysatorschicht geringer ist als die Aktivität mindestens einer in Strömungsrichtung des jeweiligen Gases gesehen stromabwärts der ersten Katalysatorschicht angeordneten zweiten Katalysatorschicht. Insbesondere wird die Aktivität der ersten Katalysatorschicht auf 5% bis 90% oder auf 10% bis 40% der Aktivität der mindestens einen zweiten Katalysatorschicht eingestellt. Die Verwendung von Katalysatorschichten mit unterschiedlicher Aktivität ist eine weitere Maßnahme zur Kontrolle des sogenannten Hotspots zur Gewährleistung eines störungsfreien Betriebs. Dies dient letztendlich auch dazu, die gewünschte Gasqualität sicher zu erreichen.

Es gibt mehrere Möglichkeiten zum Verändern der Aktivität einer Katalysatorschicht. Der Vorteil einer Verdünnung des Katalysatormaterials mit einem Inertmaterial liegt darin, dass nur eine einzige Sorte von Katalysatormaterial beim Hersteller bestellt werden muss. Die Verdünnung des Katalysatormaterials mit Inertmaterial eröffnet die Möglichkeit, den optimalen Verdünnungsgrad für ein spezielles Reaktorsystem selbst zu ermitteln. Bevorzugt hat das Verdünnungsmaterial die gleichen geometrischen Eigenschaften und die gleiche Dichte wie das eigentliche Katalysatormaterial, um Entmischungsvorgänge zu verhindern. Mit Vorteil kann ein Ruthenium-Katalysatormaterial vorzugsweise im Bereich des Hotspots auch mit einem Nickel-Katalysatormaterial verdünnt werden. In diesem Bereich herrschen naturgemäß hohe Reaktionstemperaturen, so dass dort Ruthenium-Katalysatormaterial mindestens teilweise durch ein Nickel-Katalysatormaterial ersetzt werden kann. Ferner kann die Aktivität der Katalysatorschicht durch unterschiedliche Beladungen der jeweiligen Katalysatorschicht mit dem Katalysatormaterials eingestellt werden. Dies hat den Vorteil, dass für den Betreiber einer Methanisierungsanlage der Arbeitsschritt des Verdünnens entfällt.

Diese Vorgehensweise zur Veränderung der Aktivität der Katalysatorschicht bietet sich an, wenn es experimentell sichere Ergebnisse zu den verdünnten Katalysatoren gibt oder wenn der verdünnte Katalysator einen großen Arbeitsbereich umfasst. Außerdem ist bei einem weniger beladenen Katalysator die Aktivität und damit die Reaktionstemperatur gleichmäßiger verteilt, wodurch dem Nachteil eines verdünnten Katalysators entgegengewirkt wird, bei dem ab einer gewissen Verdünnungsquote die Reaktionstemperatur sehr inhomogen wird, wodurch an jedem der einzelnen Katalysatorpartikel ein Hot Spot entstehen kann.

Vorzugsweise wird in Schritt b) in der ersten Reaktorstufe und/oder in Schritt e) in der zweiten Reaktorstufe in Strömungsrichtung des jeweiligen Gases gesehen stromaufwärts der ersten Katalysatorschicht eine dritte Katalysatorschicht verwendet, deren Aktivität größer ist als die Aktivität der ersten Katalysatorschicht und der mindestens einen zweiten Katalysatorschicht. Nach dem Start der Reaktion wird die Aktivität reduziert, um ein Durchgehen der Reaktion zu verhindern. Auch dies dient letztendlich dazu, die gewünschte Gasqualität sicher zu erreichen.

Vorzugsweise wird in Schritt b) in der ersten Reaktorstufe ein Druck von 15 bar bis 35 bar eingestellt. In Schritt e) in zweiten Reaktorstufe wird vorzugsweise ein Druck von 15 bar bis 35 bar eingestellt. Auch dies dient letztendlich dazu, die gewünschte Gasqualität sicher zu erreichen.

Vorzugsweise wird in Schritt b) und in Schritt e) als erster Wärmeträger und zweiter Wärmeträger jeweils Siedewasser verwendet. Siedewasser ist als Wärmeträger bei den herrschenden, relativ niedrigen Reaktionstemperaturen besonders vorteilhaft. Der besondere Vorteil liegt darin, dass durch eine Druckregelung eine definierte Siedetemperatur eingestellt werden kann, die zudem gleich an jedem Ort des Mantelraums ist. Umwälzeinrichtungen für das Siedewasser sind nicht erforderlich. Zudem ist der Wärmeträger Wasser leicht verfügbar und ungiftig.

Bevorzugte Weiterbildungen der Erfindung ergeben sich aus den Unteransprüchen und der nachfolgenden Beschreibung. Ausführungsbeispiele der Erfindung werden, ohne hierauf beschränkt zu sein, an Hand der Zeichnung näher erläutert. Dabei zeigt:
- Fig. 1:: eine schematisierte Darstellung eines ersten erfindungsgemäßen Reaktorsystems zur katalytischen Methanisierung eines Kohlendioxid und Wasserstoff enthaltenden Gases,
- Fig. 2:: eine schematisierte Darstellung eines zweiten erfindungsgemäßen Reaktorsystems zur katalytischen Methanisierung eines Kohlendioxid und Wasserstoff enthaltenden Gases,
- Fig. 3:: eine schematisierte Darstellung einer ersten Reaktorstufe und/oder einer zweiten Reaktorstufe des erfindungsgemäßen Reaktorsystems,
- Fig. 4a-4d:: Details der ersten Reaktorstufe und/oder einer zweiten Reaktorstufe des erfindungsgemäßen Reaktorsystems.

Die hier vorliegende Erfindung betrifft ein Verfahren sowie ein Reaktorsystem zur katalytischen Methanisierung eines Edukt-Gases, welches Kohlendioxid und Wasserstoff enthält, nämlich zur Herstellung eines Erdgassubstituts. Dabei soll ein Erdgassubstitut gewonnen werden, welches einen Kohlendioxidanteil von maximal 100 ppm(v) und einen Wasserstoffanteil von maximal 2,0 Volumen-% aufweist, vorzugsweise einen Kohlendioxidanteil von maximal 50 ppm(v) und einen Wasserstoffanteil von maximal 2,0 Volumen-%.

Dabei kommt die Erfindung insbesondere zur katalytischen Methanisierung eines Edukt-Gases zum Einsatz, welches im Wesentlichen ausschließlich aus Kohlendioxid CO₂ und Wasserstoff H₂ besteht, wobei ein Edukt-Gas, welches im Wesentlichen aus Kohlendioxid CO₂ und Wasserstoff H₂ besteht, derart ausgestaltet ist, dass die Summe aus dem Kohlendioxidanteil und dem Wasserstoffanteil im Edukt-Gas zusammen mindestens 99 Volumen-% beträgt.

Ein solches Edukt-Gas kann durch Mischung von über eine Elektrolysevorrichtung hergestelltem Wasserstoff-Gas mit Kohlendioxid-Gas gewonnen werden, wobei die Herstellung des Wasserstoff-Gases in einer Elektrolysevorrichtung insbesondere unter Nutzung regenerativ gewonnener Energie durchgeführt wird.

Auch kann die Erfindung zur katalytischen Methanisierung eines Edukt-Gases genutzt werden, welches im Wesentlichen aus Kohlendioxid CO₂, Kohlenmonoxid CO und Wasserstoff H₂ besteht, derart, dass die Summe aus dem Kohlendioxidanteil, Kohlenmonoxidanteil und dem Wasserstoffanteil im Edukt-Gases mindestens 99 Volumen-% des Edukt-Gases beträgt, bei maximal 5 Volumen-% des Kohlendioxids CO₂ durch Kohlenmonoxid ersetzt sind, sodass demnach das Verhältnis von Kohlendioxid zu Kohlenmonoxid mindestens 95 zu 5 beträgt.

Im Rest, also für die restlichen maximal 1 Volumen-% des Edukt-Gases kann das jeweilige Edukt-Gas zum Beispiel Stickstoff und/oder Sauerstoff aufweisen.

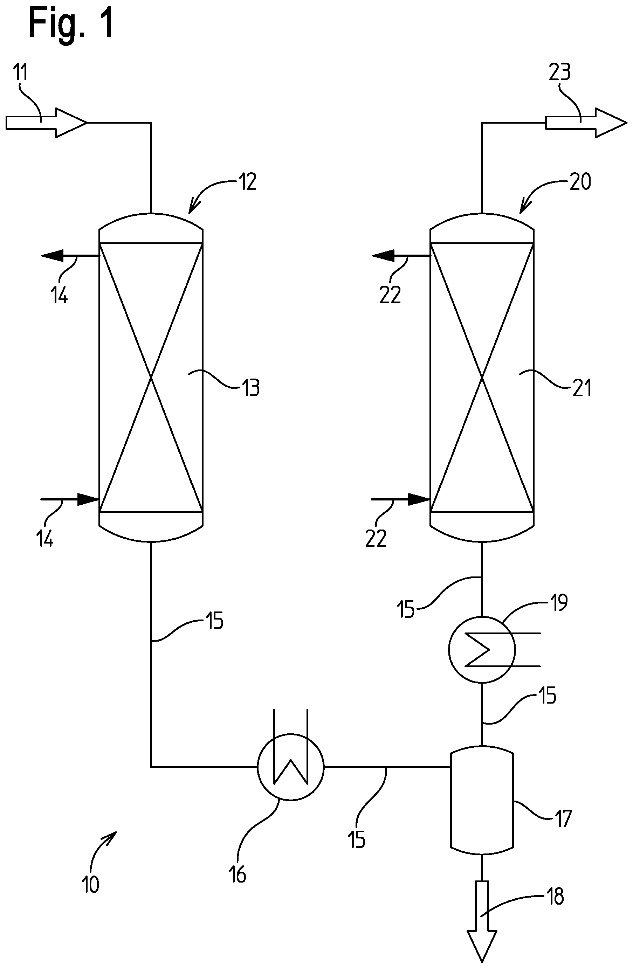

Fig. 1 zeigt stark schematisiert ein Reaktorsystem 10, welches der Methanisierung eines Edukt-Gases 11 dient. Wie bereits ausgeführt, enthält das Edukt-Gas Kohlendioxid CO₂ und Wasserstoff H₂, wobei das Edukt-Gas vorzugsweise im Wesentlichen ausschließlich aus Kohlendioxid CO₂ und Wasserstoff H₂ besteht. Das bereitgestellte Edukt-Gas 11 wird einer ersten Reaktorstufe 12 des Reaktorsystems 10 zur katalytischen Teil-Methanisierung des Edukt-Gases 11 in der ersten Reaktorstufe 12 bereitgestellt, wobei hierzu das Edukt-Gas 11 eine Katalysatorschüttung 13 der ersten Reaktorstufe 12 durchströmt und diese Katalysatorschüttung 13 der ersten Reaktorstufe 12 mithilfe eines ersten Wärmeträgers 14 gekühlt wird. Aus der ersten Reaktorstufe 12 strömt ein erstes Produkt-Gas 15 heraus, wobei das aus der ersten Reaktorstufe 12 ausströmende erste Produkt-Gas 15 mithilfe einer Kühleinrichtung 16 auf eine Temperatur abgekühlt wird, die unterhalb des Taupunkts von Wasser liegt.

Die Kühleinrichtung 16 ist in Strömungsrichtung des Edukt-Gases 11 bzw. des ersten Produkt-Gases 15 gesehen stromabwärts der ersten Reaktorstufe 12 positioniert, wobei stromabwärts dieser Kühleinrichtung 16 ein Abscheider 17 angeordnet ist, im Bereich dessen das aus dem ersten Produkt-Gas 15 auskondensierte Wasser 18 abgeleitet wird. In Strömungsrichtung des abgekühlten ersten Produkt-Gases 15 gesehen ist stromabwärts des ersten Abscheiders 17 eine Heizeinrichtung 19 angeordnet, um das erste Produkt-Gas 15, aus welchem das auskondensierte Wasser abgeleitet wurde, zu erhitzen und nachfolgend das erhitzte erste Produkt-Gas 15 einer zweiten Reaktorstufe 20 des Reaktorsystems 10 zuzuführen. In der zweiten Reaktorstufe 20 erfolgt eine katalytische End-Methanisierung des aufgeheizten ersten Produkt-Gases in einer Katalysatorschüttung 21 der zweiten Reaktorstufe 20, wobei die Katalysatorschüttung 21 der zweiten Reaktorstufe 20 durch einen zweiten Wärmeträger 22 gekühlt wird. Die zweite Reaktorstufe 20 verlässt dann ein zweites Produkt-Gas 23, welches nachfolgend in einer nicht-gezeigten zweiten Kühleinrichtung abgekühlt wird, um das zweite Produkt-Gas 23 wiederum auf eine Temperatur unterhalb des Taupunkts von Wasser abzukühlen und hierbei aus dem zweiten Produkt-Gas 23 Wasser nahezu vollständig auszukondensieren, welches dann nachfolgend in einem ebenfalls nicht-gezeigten zweiten Abscheider vom zweiten Produkt-Gas 23 getrennt und abgeleitet wird.

Demnach wird das Edukt-Gas in zwei hintereinandergeschalteten Reaktorstufen 12, 20 methanisiert, um aus dem Kohlendioxid CO₂ und Wasserstoff H₂ des Edukt-Gases 11 das zweite Produkt-Gas 23 zu gewinnen, welches dann im Wesentlichen wasserfrei ist und nahezu ausschließlich Methan CH₄ aufweist, und zwar mit einem Kohlendioxidanteil von max. 100 ppm(v), insbesondere maximal 50 ppm (v), und einen Wasserstoffanteil von max. 2,0 Vol.-%.

Um ein solches zweites Produkt-Gas mit der oben definierten Gasqualität bereitzustellen, weist das Edukt-Gas 11 in Bezug auf die Reaktion von Kohlendioxid CO₂ mit Wasserstoff H₂ einen überstöchiometrischen Wasserstoffanteil auf, der vorzugsweise einen um bis zu 0,5 % höheren Wasserstoffanteil aufweist als eine stöchiometrische Mischung aus Kohlendioxid CO₂ und Wasserstoff H₂.

Ferner werden in der ersten Reaktorstufe 12 und in der zweiten Reaktorstufe 20 Katalysatorschüttungen verwendet, die als Katalysatormaterial Ruthenium aufweisen. In der ersten Katalysatorstufe 12 und in der zweiten Katalysatorstufe 20 wird vorzugsweise eine Katalysatorschüttung 13, 21 verwendet, die einen Rutheniumanteil von jeweils zwischen 0,1 Masse-% und 5,0 Masse-% aufweist.

Innerhalb der ersten Reaktorstufe 12 und innerhalb der zweiten Reaktorstufe 20 werden jeweils Wärmeträger-Temperaturen für den jeweiligen Wärmeträger 14, 22 bereitgestellt, die zwischen 180 °C und 250 °C, vorzugsweise zwischen 200 °C und 220 °C, liegt.

Durch die Kombination zumindest der obigen Maßnahmen, also der Nutzung eines Edukt-Gases mit einem in Bezug auf die Reaktion von Kohlendioxid mit Wasserstoff überstöchiometrischen Wasserstoffanteil, durch die Verwendung von Ruthenium als Katalysatormaterial in der ersten Reaktorstufe 12 und in der zweiten Reaktorstufe 20 sowie durch die Bereitstellung der definierten Wärmeträger-Temperaturen in der ersten Reaktorstufe 12 und in der zweiten Reaktorstufe 20 kann stromabwärts der zweiten Reaktorstufe 21 das zweite Produkt-Gas 23 mit der definierten Gasqualität bereitgestellt werden, die sich durch einen Kohlendioxidanteil von maximal 100 ppm (v), insbesondere maximal 50 ppm (v), und einen Wasserstoffanteil von maximal 2,0 Vol.-% auszeichnet, und zwar insbesondere dann, wenn das Edukt-Gas im Wesentlichen aus Kohlendioxid CO₂ und Wasserstoff H₂ besteht.

In einer konkreten Verfahrensausführung wird in Fig. 1 der ersten Reaktorstufe 12 ein Edukt-Gas 11 mit einem Edukt-Gasdruck von vorzugsweise 20 bar bereitgestellt. Wie bereits ausgeführt, besteht dieses Edukt-Gas 11 im Wesentlichen ausschließlich aus Wasserstoff H₂ und Kohlendioxid CO₂ mit einem leichten Wasserstoffüberschuss.

Bei der ersten Reaktorstufe 12 handelt es sich vorzugsweise um eine isotherme Reaktorstufe, wobei innerhalb der gesamten ersten Reaktorstufe 12 die Wärmeträger-Temperatur des ersten Wärmeträgers 12 vorzugsweise zwischen 200 °C und 220 °C liegt. Dabei ist die erste Reaktorstufe 12 vorzugsweise als Rohrbündelreaktor ausgeführt. Es kann jedoch auch ein Plattenreaktor zum Einsatz kommen. Als erster Wärmeträger 14 dient dann vorzugsweise Siedewasser, wobei jedoch auch ein anderer Wärmeträger, wie Thermalöl oder eine Flüssigsalzschmelze, verwendet werden kann. In der ersten Reaktorstufe 12 wird dann als Katalysatormaterial im Wesentlichen ausschließlich Ruthenium genutzt. Die Verweilzeit des Gases in der ersten Reaktorstufe 12 ist so groß gewählt, dass sich ein nahezu thermodynamisches Reaktionsgleichgewicht bei der Wärmeträger-Temperatur von zwischen 200 °C und 220 °C einstellt und das erste Produkt-Gas 15 die erste Reaktorstufe 12 auf einem Temperaturniveau verlässt, welches wie die Temperatur des ersten Wärmeträgers 14 zwischen 200 °C und 220 °C liegt. In der ersten Reaktorstufe 12 kann bereits ein Kohlendioxid-Umsatz von über 95 %, insbesondere von über 99 %, erzielt werden. Nach dem Austritt des ersten Produkt-Gases 15 aus der ersten Reaktorstufe 12 wird das erste Produkt-Gas 15 abgekühlt, wodurch das in dem ersten Produkt-Gas 15 enthaltene Wasser auskondensiert wird. Das auskondensierte Wasser wird von der Gasphase des ersten Produkt-Gases 15 getrennt und abgeführt, wobei das hierbei gewonnene getrocknete Produkt-Gas 15 erhitzt und dann der zweiten Reaktorstufe 20 zugeführt wird. Bei der zweiten Reaktorstufe handelt es sich wiederum vorzugsweise um einen isothermen Reaktor, in dem als Katalysatormaterial wiederum Ruthenium und als zweiter Wärmeträger 22 vorzugsweise wiederum Siedewasser genutzt wird. In der zweiten Reaktorstufe 20 erfolgt die End-Methanisierung und hierbei eine nahezu vollständige Umsetzung des nach der ersten Reaktorstufe 12 noch nicht reagierten Kohlendioxids mit einem Restgehalt von weniger als 50 ppm (v) im Trockenanteil des zweiten Produkt-Gases 23. Wie bereits ausgeführt, wird auch in der zweiten Reaktorstufe 20 eine Wärmeträger-Temperatur in der Größenordnung von 200 °C bis 220 °C genutzt, das Druckniveau des der zweiten Reaktorstufe 20 zugeführten, getrockneten und erhitzten ersten Produkt-Gases 15 liegt vorzugsweise wiederum in der Größenordnung von 20 bar.

Besteht unter den obigen Bedingungen das Edukt-Gas aus 10000 Nm³/h Kohlendioxid und 40200 Nm³/h Wasserstoff, was einem Wasserstoffüberschuss von 0,5 % im Vergleich zu einer stöchiometrischen Mischung bedeutet, so reagieren in der ersten Reaktorstufe bei einem Edukt-Gas-Druck von 20 bar ca. 99 % des Kohlenstoffdioxids, sodass der Gasstrom des aus der ersten Reaktorstufe 12 austretenden ersten Produkt-Gases 15 ca. 100 Nm³/h Kohlendioxid, 600 Nm³/h Wasserstoff, 9900 Nm³/h Methan und 19800 Nm³/h Wasser besteht. Nach der Abkühlung des ersten Produkt-Gases 15 in der Kühleinrichtung 15 auf eine Temperatur von ungefähr 100 °C und nachfolgender Wasserabscheidung enthält das erste Produkt-Gas 15 stromabwärts des Abscheiders 17 nur noch 550 Nm³/h Wasser und gelangt dann nach der Erwärmung über die Heizeinrichtung 19 in den Bereich der zweiten Reaktorstufe 20. In der zweiten Reaktorstufe 22 reagieren nochmals mehr als 99,5 % des im ersten Produkt-Gas 15 vorhandenen Kohlendioxids, sodass das finale, zweite Produkt-Gas 23 im Trockenanteil eine Zusammensetzung von in etwa 98 Volumen-% Methan und 2,0 Volumen-% Wasserstoff bei einem maximalen Kohlendioxidanteil von 50 ppm (v) aufweist. In diesem konkreten Ausführungsbeispiel würde das zweite Produkt-Gas 23 dann 0,5 Nm³/h Kohlendioxid, 9999,5 Nm³/h Methan und 202 Nm³/h Wasserstoff aufweisen.

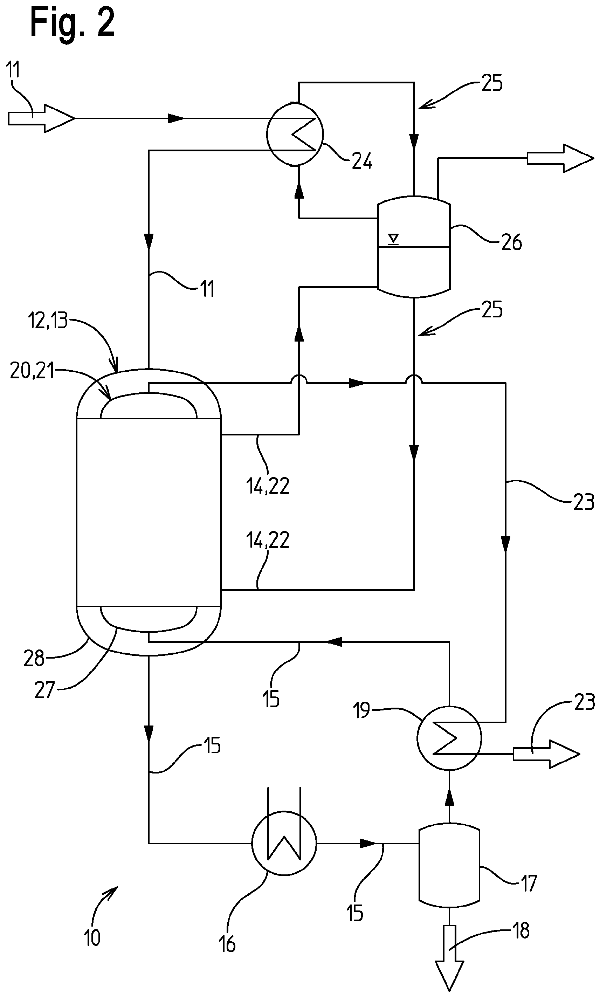

Fig. 2 zeigt ein zweites Ausführungsbeispiel eines erfindungsgemäßen Reaktorsystems 10, welches hinsichtlich seines grundsätzlichen Aufbaus dem Reaktorsystem 10 der Fig. 1 entspricht, sodass zur Vermeidung unnötiger Wiederholungen für gleiche Baugruppen gleiche Bezugsziffern verwendet werden wie in Fig. 1 und nachfolgend nur auf solche Details eingegangen wird, durch die sich das Ausführungsbeispiel der Fig. 2 vom Ausführungsbeispiel der Fig. 1 unterscheidet. Im Ausführungsbeispiel der Fig. 2 ist in Strömungsrichtung des Edukt-Gases 11 gesehen stromaufwärts der ersten Reaktorstufe 12 ein Wärmeübertrager 24 vorgesehen, der auch in einen Kreislauf 25 für den Wärmeträger 14, 22 eingebunden ist. Der Wärmeübertrager 24 wird einerseits vom Edukt-Gas 11 und andererseits von Wasserdampf durchströmt, der in den Reaktorstufen 12, 20 des Reaktorsystems 10 und in einer Dampftrommel 26 bei der Siedewasserkühlung erzeugt wurde.

Hierdurch kann eine Vorwärmung des Edukt-Gases 11 durch die bei der Methanisierung freiwerdende Reaktionswärme vorgenommen werden. Der kondensierte Dampf wird mittels einer mit Gefälle ausgeführten Rohrleitung direkt der Dampftrommel 25 zugeführt, wodurch ein sogenannter Naturumlauf entsteht. Es sind dann keine Fördereinrichtungen für den Wärmeträger 14, 22 erforderlich. Lediglich beim Anfahren des Reaktors werden ein hier nicht gezeigter Aufheizer und eine hier nicht gezeigte Umwälzpumpe benötigt.

Beim Reaktorsystem 10 der Fig. 2 ist die Reaktorstufe 20 so in die Reaktorstufe 12 integriert, dass die erste Reaktorstufe 12 die zweite Reaktorstufe 20 umgibt. Die Reaktorstufen 12 und 20 sind durch einen inneren Reaktormantel 27 voneinander getrennt und werden nacheinander durchströmt, sie befinden sich jedoch in einem gemeinsamen Wärmeträgerraum.

Ein Mantelbereich, in dem sich der als Kühlmedium dienende Wärmeträger 14, 22 befindet, ist für beide Reaktorstufen 12, 20 derselbe, weshalb in Fig. 2 die Wärmeträger-Temperaturen für die beiden Reaktorstufen 12, 20 im Unterschied zur Fig. 1 nicht unabhängig voneinander gewählt werden können.

Auch in Fig. 2 wird als Wärmeträger wiederum vorzugsweise Siedewasser genutzt, wobei in Fig. 2 zur Zirkulation des Siedewassers der oben beschriebene Naturumlauf genutzt wird. In der Dampftrommel 26 wird Wasserdampf vom flüssigen Wasser getrennt.

In Fig. 2 wird die Heizeinrichtung 19 durch einen Wärmeübertrager bereitgestellt, der vom getrockneten ersten Produkt-Gas 15 sowie vom zweiten Produkt-Gas 23 durchströmt ist. Hierdurch kann eine weitere Energieoptimierung bereitgestellt werden, da die Reaktionswärme, die in die zweite Reaktorstufe 20 verlassenden zweiten Produkt-Gas 23 vorliegt, zur Erwärmung des ersten Produkt-Gases 15 stromaufwärts der zweiten Reaktorstufe 20 genutzt werden kann.

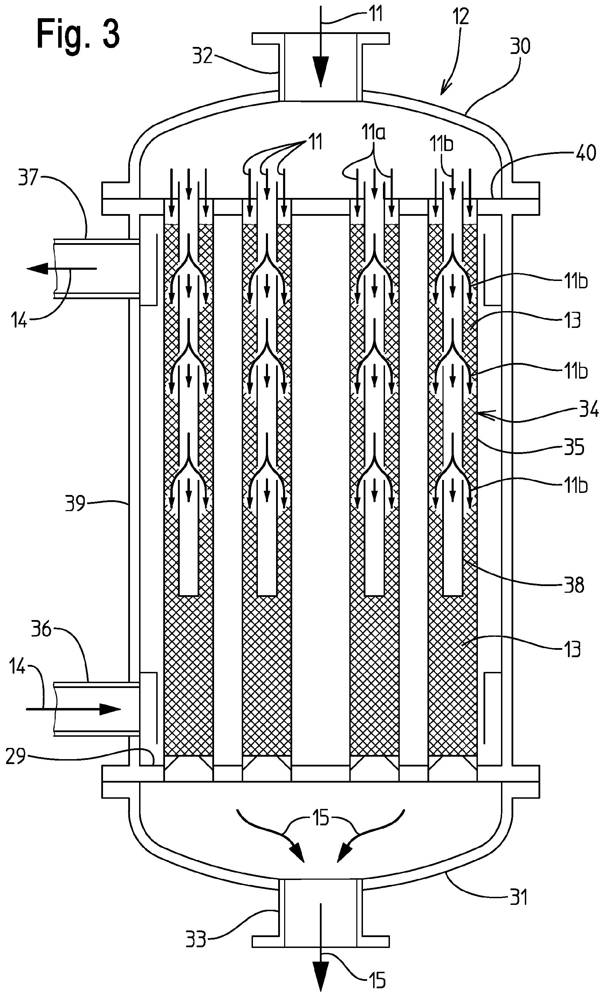

Wie bereits ausgeführt, wird in der ersten Reaktorstufe 12 sowie in der zweiten Reaktorstufe 20 vorzugsweise jeweils eine isotherme Rohrbündel-Reaktorstufe verwendet, wobei Fig. 3 einen Querschnitt durch die erste Reaktorstufe 12 der Fig. 1 zeigt. Die nachfolgend im Zusammenhang mit Fig. 3 beschriebenen Details der ersten Reaktorstufe 12 sind für die ersten Reaktorstufe 12 besonders bevorzugt, können auch in der zweiten Reaktorstufe 20 zum Einsatz kommen.

Die Reaktorstufe 12 der Fig. 3 verfügt über ein Reaktorgehäuse aus einem zylindrischen Reaktormantel 39, einem oberen Rohrboden 40, einem unteren Rohrboden 29, einer oberen Reaktorhaube 30 und einer unteren Reaktorhaube 31. Im Bereich der oberen Reaktorhaube 30 ist ein Zulauf 32 für das Edukt-Gas 11 ausgebildet, im Bereich der unteren Reaktorhaube 31 ist ein Ablauf 33 für das erste Produkt-Gas 15 ausgebildet. Der zylindrische Reaktormantel 39 nimmt ein Bündel 34 aus Reaktionsrohren 35 auf, die von dem Edukt-Gas 11 durchströmt sind. Zur Kühlung werden die Reaktionsrohre 35 vom Wärmeträger 14 umströmt, wobei der Wärmeträger 14 über einen Vorlauf 36 der ersten Reaktorstufe 12 zugeführt und über einen Rücklauf 37 von der ersten Reaktorstufe 12 abgeführt wird. Die Reaktionsrohre 35 sind mit der jeweiligen Katalysatorschüttung 13 befüllt, wobei die Katalysatorschüttung 13 als Katalysatormaterial Ruthenium aufweist. Wie bereits ausgeführt, sind die mit der Katalysatorschüttung 13 befüllten Reaktionsrohre 35 vom Edukt-Gas 11 durchströmt und vom Wärmeträger 14 umströmt.

Im Ausführungsbeispiel der Fig. 3 wird der Edukt-Gasstrom 11 in Teilströme 11a, 11b aufgeteilt, die an unterschiedlichen Positionen in die jeweilige Katalysatorschüttung 13 eingeleitet werden. So zeigt Fig. 3, dass der Teilstrom 11a unmittelbar am Eintritt der Reaktionsrohre 15 in die Katalysatorschüttung 13 einritt, wohingegen der Teilstrom 11b an unterschiedlichen Positionen entlang der Reaktionsrohre 15 in die Katalysatorschüttung 13 eintritt. Dies wird in Fig. 3 durch Dosierrohre 38 gewährleistet.

Die Dosierrohre 38 erstrecken sich koaxial zum jeweiligen Reaktionsrohr 35, wobei jedoch das jeweilige Dosierrohr 38 keine Katalysatorschüttung 13 enthält, sondern lediglich ein vom jeweiligen Dosierrohr 38 und jeweiligen Reaktionsrohr 35 definierter Ringkanal sowie derjenige Abschnitt des Reaktionsrohrs 35, in den sich das jeweilige Dosierohr 38 nicht erstreckt.

So erstreckt sich das jeweilige Dosierrohr 38 um eine definierte Länge in das jeweilige Reaktionsrohr 35 hinein, jedoch nicht über die gesamte Länge des jeweiligen Reaktionsrohrs 35. Das jeweilige Dosierrohr 38 weist entlang seiner Längserstreckung Öffnungen auf, über die dann der Teilstrom 11b an unterschiedlichen axialen Positionen des jeweiligen Dosierrohrs 38 in die Katalysatorschüttung 13 eintreten kann, und zwar in die Katalysatorschüttung, die in dem Ringraum angeordnet ist, der radial innen vom jeweiligen Dosierrohr 38 und radial außen vom jeweiligen Reaktionsrohr 35 begrenzt ist.

Über das jeweilige Dosierrohr 38 kann eine Hotspot-Temperatur reduziert werden. Dies liegt daran, dass das Edukt-Gas 11 in die Teilströme 11a und 11b aufgeteilt wird, wobei der Teilstrom 11a unmittelbar in den Ringspalt zwischen dem jeweiligen Dosierrohr 38 und dem jeweiligen Reaktionsrohr 35 gelangt und die Katalysatorschüttung 13 durchströmt, während der Teilstrom 11b zunächst das leere Dosierrohr 38 durchströmt. Wie bereits ausgeführt, sind entlang der Achse des jeweiligen Dosierrohrs 38 mehrere Öffnungen, insbesondere mehrere radiale Bohrungen, angeordnet, durch die dann der Teilstrom 11b in die Katalysatorschüttung 13 eintreten kann. Durch diese Strömungsführung wird der Reaktionsumsatz entlang der Achse des Reaktionsrohrs 35 vergleichmäßigt und konzentriert sich nicht auf den oberen Teil des Reaktionsrohrs 35, wodurch die Wärmefreisetzung bei der Methanisierung entlang des jeweiligen Reaktionsrohrs besser verteilt wird. Dies resultiert dann in einer niedrigeren Hotspot-Temperatur. Ferner reduziert das jeweilige Dosierrohr 38 den Strömungsquerschnitt der Katalysatorschüttung 13 im jeweiligen Reaktionsrohr 35 auf den Querschnitt des Ringraums zwischen dem jeweiligen Reaktionsrohr 35 und dem jeweiligen Dosierrohr 38, wodurch die sich einstellende Reaktionswärme reduziert wird.

Schließlich wird quer zur Strömungsrichtung des Edukt-Gases 11 der Wärmeableitungsweg verkürzt, nämlich auf die Abmessung des Ringspalts zwischen dem jeweiligen Dosierrohr 38 und dem jeweiligen Reaktionsrohr 35, sodass nicht nur weniger Reaktionswärme entsteht, sondern diese auch schneller abgeleitet werden kann.

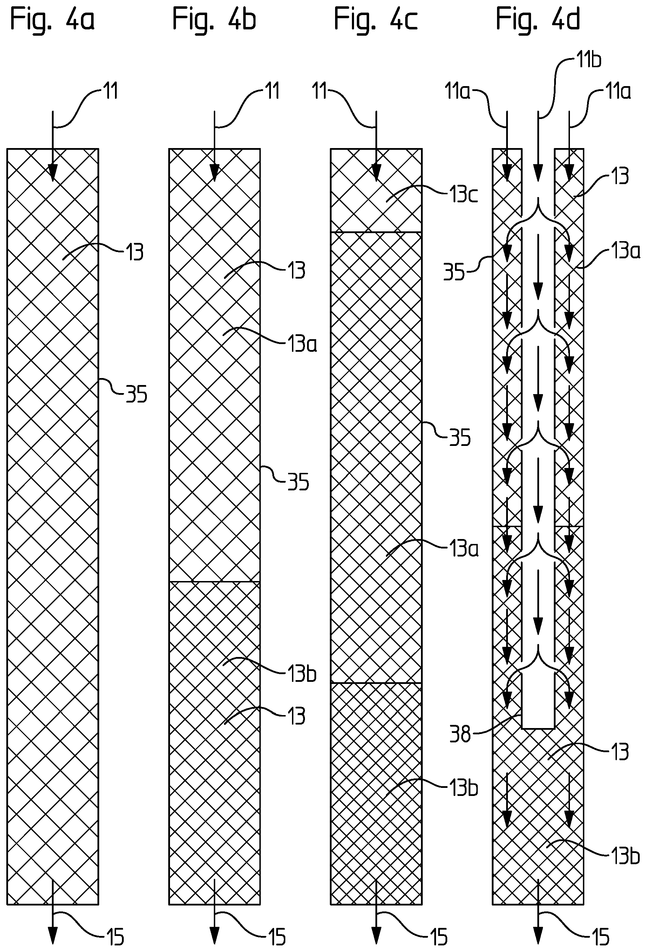

Fig. 4a, 4b, 4c und 4d zeigen jeweils ein Reaktionsrohr 35, welches mit einer jeweiligen Katalysatorschüttung 13 gefüllt ist, wobei im Ausführungsbeispiel der Fig. 4d ebenso wie in Fig. 3, jedoch im Unterschied zu den Ausführungsbeispielen der Fig. 4a, 4b und 4c, innerhalb des Reaktionsrohrs 35 ein Dosierrohr 38 angeordnet ist, welches selbst nicht mit der Katalysatorschüttung 13 befüllt ist, sondern lediglich der Teil des Reaktionsrohrs 35, in dem sich einerseits das Dosierrohr 38 nicht hinein erstreckt und der andererseits vom Ringraum zwischen dem Dosierrohr 38 und dem jeweiligen Reaktionsrohr 35 definiert ist. Im Unterschied zur Fig. 3 besteht in Fig. 4d die Katalysatorschüttung 13 aus zwei Katalysatorschichten 13a, 13b mit unterschiedlicher Aktivität. In Fig. 4d weist dabei die erste Katalysatorschicht 13a eine geringere Aktivität als die zweite Katalysatorschicht 13b auf, wobei die zweite Katalysatorschicht 13b stromabwärts der ersten Katalysatorschicht 13a angeordnet ist.

Demgegenüber zeigt Fig. 4a ein Reaktionsrohr 35 mit einer Katalysatorschüttung 13, wobei das Reaktionsrohr 35 entlang seiner gesamten axialen Länge mit der gleichen Katalysatorschüttung 13 befüllt ist, die also entlang der axialen Länge des Reaktionsrohrs 35 eine gleiche Aktivität aufweist.

Im Ausführungsbeispiel der Fig. 4b ist innerhalb des Reaktionsrohrs 35 eine Katalysatorschüttung 13 mit den beiden Katalysatorschichten 13a, 13b gezeigt, wobei in Fig. 4b wie in Fig. 4d die erste Katalysatorschicht 13a eine geringere Aktivität aufweist als die zweite Katalysatorschicht 13b, die stromabwärts der ersten Katalysatorschicht 13a angeordnet ist.

In Fig. 4c ist ferner stromaufwärts der ersten Katalysatorschicht 13c eine dritte Katalysatorschicht 13c vorhanden, deren Aktivität größer ist als die Aktivität der ersten Katalysatorschicht 13a sowie der zweiten Katalysatorschicht 13b.

Während also in Fig. 4a entlang der gesamten Länge des dort gezeigten Reaktionsrohrs 35 die Katalysatorschüttung 13 eine einheitliche Reaktionsgeschwindigkeit bereitstellt, variieren in Fig. 4b, 4c und 4d die Reaktionsgeschwindigkeiten in Axialrichtung bzw. Längsrichtung des jeweiligen Reaktionsrohrs 35, und zwar durch entsprechend hinsichtlich ihrer Aktivität unterschiedliche Katalysatorschichten 13a, 13b, 13c. Weist, wie in Fig. 4b und 4d gezeigt, die erste Katalysatorschicht 13a eine geringere Aktivität auf als die zweite Katalysatorschicht 13b, so wird im Bereich der ersten Katalysatorschicht 13a die Reaktionsgeschwindigkeit gedämpft, sodass hier eine geringere Wärmefreisetzung erfolgt.

In Fig. 4c weist die dritte Katalysatorschicht 13c eine höhere Aktivität auf als die erste Katalysatorschicht 13a. Die dritte Katalysatorschicht 13c, die stromaufwärts der ersten Katalysatorschicht 13a angeordnet ist, weist dann vorzugsweise eine geringere Länge bzw. Dicke als die anderen Katalysatorschichten 13a, 13b auf. Dies dient dann dazu, bereits im in Strömungsrichtung des Edukt-Gases 11 gesehen vorderen Abschnitt des jeweiligen Reaktionsrohrs 35 durch die hohe Aktivität der dritten Katalysatorschicht 13c einen steilen Temperaturanstieg zu gewährleisten, wobei jedoch die Temperatur nicht über eine maximal zulässige Grenztemperatur hinausgeht. Die sich an die dritte Katalysatorschicht 13c anschließende erste Katalysatorschicht 13a dämpft den Temperaturanstieg in Fig. 4c ab, sodass auch in der ersten Katalysatorschicht 13c die maximal zulässige Grenztemperatur nicht überschritten wird.

Auch in den Fig. 4a, 4b und 4c kann ein Dosierrohr 38 zum Einsatz kommen.

Die Aktivität der Katalysatorschichten kann auf verschiedene Art und Weise modifiziert werden. Zum einen kann die Katalysatorschicht, die als Katalysatormaterial Ruthenium aufweist, zur Reduzierung der Aktivität mit Inert-Material verdünnt werden. Alternativ oder zusätzlich zu der Verdünnung des Katalysators mit Inert-Material kann die Katalysatorschicht mit Nickel-Katalysatormaterial verdünnt werden. Ferner können die einzelnen Katalysatorschichten unterschiedliche Beladungen mit Katalysatormaterial aufweisen, also unterschiedliche Massen-Prozente an Katalysatormaterial.

Vorteile der Erfindung ergeben sich insbesondere aus der Verwendung von Ruthenium als Katalysatormaterial. Ruthenium kann in reduzierter (elementarer) Form beschafft und transportiert werden. Im Vergleich dazu kann Nickel nur als Nickeloxid sicher gehandelt und transportiert werden. Um einen Nickelkatalysator in einer Methanisierungsanlage zu verwenden, muss dieser zunächst einer aufwändigen In-Situ-Aktivierungsprozedur, bei der das Nickeloxid mit Wasserstoff zu elementarem Nickel reduziert wird, unterzogen werden. Diese Prozedur entfällt durch die Verwendung von Ruthenium als Katalysatormaterial. Darüber hinaus ist Ruthenium im Gegensatz zu Nickel nicht pyrophor, nicht toxisch und nicht kanzerogen, was den Umgang mit dem Katalysatormaterial einfacher und sicherer für das Personal gestaltet. Zudem ist Ruthenium im Gegensatz zu Nickel nicht als umweltgefährdend eingestuft. Da Ruthenium erst ab ca. 800°C mit Sauerstoff reagiert, kann eine erhöhte Menge an Sauerstoff-Verunreinigung im Edukt-Gas zugelassen werden, ohne dass ein sogenannter Deoxidationsreaktor, welcher mit einem Edelmetallkatalysator bestückt ist, der ersten Reaktorstufe vorgeschalten werden müsste. Diese Problematik ist vor allem bei Power-to-Gas Anlagen relevant, da die meisten Herstellungsprozesse von Wasserstoff aus elektrischer Energie (Elektrolyse) einen Wasserstoffstrom liefern, der mit bis zu 0,5 Vol.-% Sauerstoff kontaminiert wird. Im Vergleich zu Nickel besteht darüber hinaus bei niedrigen Prozesstemperaturen keine Gefahr der irreversiblen Reaktion des Katalysatormaterials mit Kohlenstoffmonoxid zu flüchtigem und hochgiftigem Nickeltetracarbonyl.

### Bezugszeichenliste

- 10: Reaktorsystem
- 11: Edukt-Gas
- 11a: Teilstrom
- 11b: Teilstrom
- 12: erste Reaktorstufe
- 13: Katalysatorschüttung
- 13a: Katalysatorschicht
- 13b: Katalysatorschicht
- 13c: Katalysatorschicht
- 14: erster Wärmeträger
- 15: erstes Produkt-Gas
- 16: Kühleinrichtung
- 17: Abscheider
- 18: Wasser
- 19: Heizeinrichtung
- 20: zweite Reaktorstufe
- 21: Katalysatorschüttung
- 22: zweiter Wärmeträger
- 23: zweites Produkt-Gas
- 24: Wärmeübertrager
- 25: Kreislauf
- 26: Dampftrommel
- 27: innerer Reaktormantel
- 28: äußerer Reaktormantel
- 29: unterer Rohrboden
- 30: obere Reaktorhaube
- 31: untere Reaktorhaube
- 32: Zulauf
- 33: Ablauf
- 34: Bündel
- 35: Reaktionsrohr
- 36: Vorlauf
- 37: Rücklauf
- 38: Dosierrohr
- 39: Reaktormantel
- 40: oberer Rohrboden

## Patentansprüche

1. Verfahren zur katalytischen Methanisierung eines Kohlendioxid und Wasserstoff enthaltenden Edukt-Gases, mit folgenden Schritten:
a) Bereitstellen des Kohlendioxid und Wasserstoff enthaltenden Edukt-Gases,
b) katalytische Teil-Methanisierung des Edukt-Gases in einer ersten Reaktorstufe, deren Katalysatorschüttung von einem ersten Wärmeträger gekühlt wird,
c) Abkühlen eines aus der ersten Reaktorstufe ausströmenden ersten Produkt-Gases auf eine Temperatur unterhalb des Taupunkts von Wasser und Ableiten des hierbei kondensierenden Wassers,
d) Aufheizen des ersten Produkt-Gases, aus welchem das im Schritt c) auskondensierte Wasser abgeleitet wurde,
e) katalytische End-Methanisierung des in Schritt d) aufgeheizten ersten Produkt-Gases in einer zweiten Reaktorstufe, deren Katalysatorschüttung von einem zweiten Wärmeträger gekühlt wird,
f) Abkühlen eines aus der zweiten Reaktorstufe ausströmenden zweiten Produkt-Gases auf eine Temperatur unterhalb des Taupunkts von Wasser und Ableiten des hierbei kondensierten Wassers,
**dadurch gekennzeichnet, dass**
zur Gewinnung eines zweiten Produkt-Gases, welches einen Kohlendioxidanteil von maximal 100 ppm(v) und einen Wasserstoffanteil von maximal 2,0 Volumen-% aufweist,
in Schritt a) ein Edukt-Gas bereitgestellt wird, das in Bezug auf die Reaktion von Kohlendioxid mit Wasserstoff einen überstöchiometrischen Wasserstoffanteil aufweist,
in Schritten b) und e) in der ersten und in der zweiten Reaktorstufe die jeweilige Katalysatorschüttung als Katalysatormaterial Ruthenium aufweist,
in Schritten b) und e) innerhalb der ersten Reaktorstufe und innerhalb der zweiten Reaktorstufe jeweils eine Wärmeträger-Temperatur bereitgestellt wird, die zwischen 180°C und 250°C liegt.

2. Verfahren nach Anspruch 1,
**dadurch gekennzeichnet, dass**
dasselbe der Gewinnung eines zweiten Produkt-Gases dient, welches einen Kohlendioxidanteil von maximal 50 ppm(v) und einen Wasserstoffanteil von maximal 2,0 Volumen-% aufweist.

3. Verfahren nach Anspruch 1 oder 2,
**dadurch gekennzeichnet, dass**
in Schritt b) in der ersten Reaktorstufe und in Schritt e) in der zweiten Reaktorstufe jeweils eine Katalysatorschüttung verwendet wird, die einen Rutheniumanteil von jeweils zwischen 0,1 Masse-% und 5,0 Masse-% aufweist.

4. Verfahren nach Anspruch 1, 2 oder 3,
**dadurch gekennzeichnet, dass**
das in Schritt a) bereitgestellte Edukt-Gas in Bezug auf die Reaktion von Kohlendioxid mit Wasserstoff einen um bis zu 0,5 % höheren Wasserstoffanteil als eine stöchiometrische Mischung aufweist.

5. Verfahren nach einem der Ansprüche 1 bis 4,
**dadurch gekennzeichnet, dass**
das in Schritt a) bereitgestellte Edukt-Gas im Wesentlichen aus Kohlendioxid und Wasserstoff besteht, derart, dass die Summe aus dem Kohlendioxidanteil und dem Wasserstoffanteil im Edukt-Gases mindestens 99 Volumen-% des Edukt-Gases beträgt, oder
das in Schritt a) bereitgestellte Edukt-Gas im Wesentlichen aus Kohlendioxid, Kohlenmonoxid und Wasserstoff besteht, derart, dass die Summe aus dem Kohlendioxidanteil, Kohlenmonoxidanteil und dem Wasserstoffanteil im Edukt-Gases mindestens 99 Volumen-% des Edukt-Gases beträgt.

6. Verfahren nach einem der Ansprüche 1 bis 5,
**dadurch gekennzeichnet, dass**
in Schritt b) in der ersten Reaktorstufe und in Schritt e) in der zweiten Reaktorstufe die Wärmeträger-Temperatur jeweils zwischen 200°C und 230°C oder zwischen 200°C und 220°C liegt.

7. Verfahren nach einem der Ansprüche 1 bis 6,
**dadurch gekennzeichnet, dass**
das nach Schritt c) vorliegende erste Produkt-Gas einen Wasserdampfgehalt von maximal 30 Volumen-% aufweist, bevorzugt einen Wasserdampfgehalt von zwischen 5 Volumen-% und 15 Volumen-%.

8. Verfahren nach einem der Ansprüche 1 bis 7,
**dadurch gekennzeichnet, dass**
in Schritten b) und e) als Reaktorstufe jeweils eine isotherme Rohrbündel-Reaktorstufe verwendet wird, die jeweils ein Bündel aus mit der Katalysatorschüttung befüllten Reaktionsrohren aufweist, die vom Wärmeträger gekühlt werden.

9. Verfahren nach einem der Ansprüche 1 bis 8,
**dadurch gekennzeichnet, dass**
in Schritt b) in der ersten Reaktorstufe und/oder in Schritt e) in der zweiten Reaktorstufe das in die jeweilige Reaktorstufe einströmende Gas in mindestens zwei Teilströme aufgeteilt wird, die an unterschiedlichen Positionen in die jeweilige Katalysatorschüttung eingeleitet werden.

10. Verfahren nach einem der Ansprüche 1 bis 9,
**dadurch gekennzeichnet, dass**
in Schritt b) in der ersten Reaktorstufe und/oder in Schritt e) in der zweiten Reaktorstufe eine Katalysatorschüttung verwendet wird, die mindestens zwei Katalysatorschichten unterschiedlicher Aktivität aufweist, wobei die Aktivität einer ersten Katalysatorschicht geringer ist als die Aktivität mindestens einer in Strömungsrichtung des jeweiligen Gases gesehen stromabwärts der ersten Katalysatorschicht angeordneten zweiten Katalysatorschicht.

11. Verfahren nach Anspruch 10,
**dadurch gekennzeichnet, dass**
die Aktivität der ersten Katalysatorschicht auf 5% bis 90% oder auf 10% bis 40% der Aktivität der mindestens einen zweiten Katalysatorschicht eingestellt wird.

12. Verfahren nach Anspruch 10 oder 11,
**dadurch gekennzeichnet, dass**
die Aktivität der jeweiligen Katalysatorschicht durch Verdünnung des Katalysatormaterials mit Inertmaterial und/oder durch Verdünnung des Katalysatormaterials mit einem Nickel-Katalysatormaterial und/oder durch unterschiedliche Beladungen der jeweiligen Katalysatorschicht mit dem Katalysatormaterial eingestellt wird.

13. Verfahren nach Anspruch 10, 11 oder 12,
**dadurch gekennzeichnet, dass**
in Schritt b) in der ersten Reaktorstufe und/oder in Schritt e) in der zweiten Reaktorstufe in Strömungsrichtung des jeweiligen Gases gesehen stromaufwärts der ersten Katalysatorschicht eine dritte Katalysatorschicht verwendet wird, deren Aktivität größer ist als die Aktivität der ersten Katalysatorschicht und der mindestens einen zweiten Katalysatorschicht.

14. Verfahren nach einem der Ansprüche 1 bis 13,
**dadurch gekennzeichnet, dass**
in Schritt b) in der ersten Reaktorstufe ein Edukt-Gas-Druck und/oder in Schritt e) in der zweiten Reaktorstufe ein Druck von 15 bar bis 35 bar eingestellt wird.

15. Verfahren nach einem der Ansprüche 1 bis 14,
**dadurch gekennzeichnet, dass**
in Schritt b) und in Schritt e) als erster Wärmeträger und zweiter Wärmeträger jeweils Siedewasser verwendet wird.

16. Reaktorsystem zur Durchführung des Verfahrens nach einem der Ansprüche 1 bis 15, mit
einer ersten Reaktorstufe, die zur katalytischen Teil-Methanisierung des Edukt-Gases unter Kühlung der Katalysatorschüttung durch einen ersten Wärmeträger eingerichtet ist, wobei die Katalysatorschüttung der ersten Reaktorstufe als Katalysatormaterial Ruthenium aufweist,
einem ersten Kondensator stromabwärts der ersten Reaktorstufe, der zum Abkühlen des aus der ersten Reaktorstufe ausströmenden ersten Produkt-Gases auf eine Temperatur unterhalb des Taupunktes von Wasser eingerichtet ist,
einem ersten Abscheider stromabwärts des ersten Kondensators, der zum Ableiten des aus dem ersten Produkt-Gas auskondensierten Wassers eingerichtet ist,
einer Heizeinrichtung stromabwärts des ersten Abscheiders, die zum Aufheizen des ersten Produkt-Gases eingerichtet ist,
einer zweiten Reaktorstufe stromabwärts der Heizeinrichtung, die zur katalytischen End-Methanisierung des ersten Produkt-Gases unter Kühlung der Katalysatorschüttung durch einen zweiten Wärmeträger eingerichtet ist, wobei die Katalysatorschüttung der zweiten Reaktorstufe als Katalysatormaterial Ruthenium aufweist,
einem zweiten Kondensator stromabwärts der zweiten Reaktorstufe, der zum Abkühlen des aus der zweiten Reaktorstufe ausströmenden zweiten Produkt-Gases auf eine Temperatur unterhalb des Taupunkts von Wasser eingerichtet ist,
einem zweiten Abscheider stromabwärts des zweiten Kondensators, der zum Ableiten des aus dem zweiten Produkt-Gas auskondensierten Wassers eingerichtet ist,
einer Mischeinrichtung, die eingerichtet ist, ein Edukt-Gas bereitzustellen, das in Bezug auf die Reaktion von Kohlendioxid mit Wasserstoff einen überstöchiometrischen Wasserstoffanteil aufweist,
einem Regler, der eingerichtet ist, in der ersten und zweiten Reaktorstufe jeweils eine Wärmeträger-Temperatur zwischen 180°C und 250°C einzustellen.
